# EUROPEAN PATENT APPLICATION

(11) **EP 2 450 052 A2**
(43) Date of publication of application: **09.05.2012**
(21) Application number: 12153095.0
(22) Date of filing: 14.08.2008
(51) Int. Cl.: A61K 39/00, C12N 5/0784

(54) **Dendritic cells**

(30) Priority: 16.08.2007 EP 07450139
(62) Divisional of application: 08787211.5
(71) Applicant: Cell Med Research GMBH, 3502 Krems-Lerchenfeld (AT)
(72) Inventor: Rubiolo, Christina, 1210 Wien (AT)
(74) Representative: Gassner, Birgitta

(57) **Abstract**

The present invention relates to dendritic cells loaded with at least one nucleic acid molecule encoding a tumor associated antigen or fragment thereof and at least one proteic molecule being a tumor associated antigen or fragment thereof and methods for manufacturing the same.

## Description

The present invention relates to dendritic cells and their use.

Dendritic cells (DCs) are bone-marrow-derived antigen-presenting cells (APCs) that play a pivotal role in both induction and regulation of the immune response. It has been described that the *in vitro* generation and manipulation of human DCs can be particularly effective to stimulate the immune system against cancer, being a new powerful tool in the fight against tumor. Indeed, it has been shown that in patients suffering from different types of cancer such as but not only breast, ovarian, head and neck, colorectal and renal tumor, hepatocellular carcinoma and malignant melanoma, the incapability of the host's immune system to fight efficiently the cancer establishment strictly correlates with diminished DC function. These changes have been mainly detected in blood, in cells infiltrating cancers and in lymphonodes (LNs). In addition, it has been documented that most chemotherapies deeply impair DC function, whereas the capability of patients' T-cell to drive an effective immune response is comparable with that observe in healthy people. This observation implies that the openness to tumor establishment displayed by cancer patients' immune system is mostly due to the presence of functional defects in the DCs such as maintenance of an immature phenotype and/or incorrect balance between IL-12 and IL-10 secretion. Accordingly, it has been shown that the serum levels of IL-10 levels are significantly higher in patients suffering from cancer than in healthy age- and sex-matched controls. IL-10 has been shown to have a significant inhibitory effect on several aspects of DC function, such as the expression of costimulatory molecules and the ability to synthesize IL-12. Importantly, IL-10-treated DCs become tolerogenic. In addition, it has been reported that in patients with progressively tumor growing, the DCs are both immature and switched off. Since the antitumor effect of DCs depends on their level of activation and maturation, it is likely that the failure of induction of a tumor-specific T-cell driven cytotoxic response depends on the wrong balance between IL-10 and IL-12 as well as on the absence of maturing markers, required to elicit the expected T-cell activation. Indeed the immune suppressive function of IL-10 can be modulated by a simultaneous production of IL-12. For example, it has been shown that monocyte-derived mature DCs commonly secrete both IL-12 and IL-10 upon stimulation with CD40L. In this case, the coupled secretion of IL-10 and IL-12 induces a more effective immune response against cancer than that observed upon secretion of IL-12 alone. In the presence of mild-high levels of IL-12 production, IL-10 secretion promotes the activation of the T-helper cells (CD4⁺ T-cells) and therefore triggers the adaptive arm of the immune system.

One of the main goals of the current immune therapy strategies is to activate efficiently the host's immune system against cancer. Among them, the use of *in vitro* cultured and modified DCs and T-cells are the most common methods. Although many small *in vitro* studies, animal models as well as clinical trial have shown partial success, especially regarding the triggering of a strong cytotoxic response mediated by CD8⁺ T-cells, they all failed to show the establishment of a powerful adaptive response, which would be able to fight any tumor relapse even after years upon completion of the therapy.

The most common approach to use DCs for vaccines is to prepare large numbers of autologous mature myeloid DC (MDCs) ex vivo, load them with cancer-specific antigen(s), and inject them back into the subject. Since the rate of transdifferentiation from monocytes to mature DCs is low, it is important to gain an amount of DC precursors compatible with large-scale production of mature DCs. This goal is usually achieved alternatively by (1) differentiating DCs from leukapheresis/elutriation-derived monocytes with GM-CSF and IL-4 or with GM-CSF and IL-13 or (2) directly isolating DCs from leukapheresis products by density gradient centrifugation or with commercially available closed systems, based on the use of immunomagnetic beads.

Once transdifferentiated from monocytes, the immature DCs (iDCs) should be loaded and matured *in vitro.* While the loading step can be performed according to several methods as single native or modified protein antigen, mRNA, cDNA, tumor lysate, etc..., the maturation is usually performed by means of addition of one of the following cocktails: (a) IL-1β, IL-6, TNF-α, and PGE2; (b)LPS and IFN-γ; (c) Ribomunyl and IFN-γ. Although in the art it is observed that DCs matured with a cocktail including PGE2 still express CCR7 and induce Th1 as well as CD8⁺ T-cell responses, they fail to secrete detectable bioactive IL-12p70, in contrast to DCs matured with LPS/Ribomunyl and IFN-γ.

In Zhou Y. et al. (J. Immu. Thera. 25 (2002): 289-303) methods for loading dendritic cells with tumor antigens are described. The authors of this publication report that the loading may occur with peptides or proteins or alternatively with nucleic acid molecules encoding said peptides and proteins.

In Onaitis M. et al. (Surg. Oncol. Clin. N. Am. 11 (2002): 645-660) serveral methods for loading dendritic cells with antigens are disclosed.

In the rieview article of Osada T. et al. (Int. Rev. Immunol. 25 (2006): 377-413) immunotherapies based on dendritic cells are described. The dendritic cells used in these methods can be loaded with tumor associated antigens.

In Rammensee H-G (Immunol. Cell Biol. 84 (2006): 290-294) the advantages of dendritic cells loaded either with peptides or with RNA molecules for vaccination purposes are described.

In the WO 2006/020889 loading of dendritic cells with mRNA molecules coding for TERT or fragments thereof is disclosed.

In Ciesielski M.J. et al. (Cancer Immunmol. Immunother. 55 (2006): 1491-1503) a method for producing dendritic cells loaded with DNA coding for survivin is disclosed. Such cells are capable to express in said dendritic cells survivin.

In Otto K. et al. (Vaccine 23 (2005): 884-889) the usability of dendritic cells loaded with epitopes of survivin for tumor vaccination was investigated.

In Hsu A.K.W. et al. (Biol. Blood Marrow Transplant. 12 (2006): 855-867) dendritic cells loaded with mRNA molecules coding for survivin are disclosed.

In Morse M.A. et al. (Cancer Res. 58 (1998): 2965-2968) loading of dendritic cells with peptides which are derived from the tumor associated antigen CEA or its coding RNA is disclosed.

Also in Nair S.K. et al. (Int. J. Cancer 82 (1999): 121-124) dendritic cells loaded with the tumor associated antigen CEA are described.

In Fuessel S. et al., (Prostate 66 (2006): 811-821) clinical investigations are disclosed in which dendritic cells which have been loaded with PSA, prostate specific membrane antigen (PSMA), survivin, prostein and transient receptor potential p8 (trp-p8) are administered to 8 patients.

All these documents show loading methods based either on protein antigen(s) or mRNA/cDNA antigen(s).

It is an object of the present invention to provide dendritic cells showing enhanced immunologic properties.

These DCs should preferably show (a) at least a 80% reduced phagocyting capability in comparison with iDCs; (b) capability to migrate in response to stimuli (such as but not only CCL19 and/or CD40L;(c) a mature phenotype i.e. high surface expression of CD80, CD86, CD40, CD83, HLA-ABC and HLA-DR; (d) mild to high levels of IL-12 secretion with or without IL-10; (e) surface expression of the loaded antigen(s)upon stimulation with CD40L.

The activation and establishment of both the short-term immune response by CD8⁺ T-cells (cytotoxic) and of the long-term immune response by CD4⁺ T-cells (adaptive) is a further goal to be achieved by these DCs.

The present invention relates to a dendritic cell loaded with at least one nucleic acid molecule encoding a tumor associated antigen protein or fragment thereof and at least one tumor associated antigen protein or fragment thereof.

The antigen encoded by the at least one nucleic acid molecule will be translated into an endogenous protein and therefore processed by the DCs and exposed on their cell membrane complexed to MHC-I. In this way, it triggers the cytotoxic response, due to the fact that MHC-I exposes antigens exclusively to CD8⁺. On the other side, the protein antigen loaded into DCs will be processed by said DCs as foreign protein antigen and exposed on their cell membrane complexed to MHC-II. In this way, it triggers the adaptive response, due to the fact that MHC-II exposes antigens exclusively to CD4⁺. Accordingly, the double loading with at least a nucleic acid molecule and a protein tumor antigen ensures: (a) a short-term response of the immune system by activating the cytotoxicity through T-cytotoxic, NK and NKT upon recognition of the antigen exposed through MHC-I; (b) the stability of the cytotoxic response by CD4⁻-dependent sustained CD8⁺ activation upon recognition of the antigen exposed through MHC-II; (c) the activation of the antibody mediated memory response by the cascade activation of the T-memory and B-cells upon recognition of the antigen exposed through MHC-II.

Briefly, the double loading strategy (a) activates on the short time the cytotoxic response, required for the first, efficient tumor clearance; (b) expands the timespan of the cytotoxic response, which is commonly transitional and therefore not highly efficient by activating the T-helper cells; (c) promotes the generation and sustains the establishment of the adaptive immune response, which needs a long time but differently for the cytotoxic activates the immune system against tumor even after years upon therapy completion.

The DCs of the present invention are preferably loaded (a) with universal Tumor Associated Antigens (or uTAAs, which are present in more than 2, preferably more than 5, more preferably more than 10, even more preferably more than 20, in particular more than 30, different types of tumors) and/or (b) with type-specific tumor antigens (TAAs; characteristic of a single tumor type and/or a single tumor family type). For example, this method can be applied to a combined use of the two most common uTAAs: Survivin and TElomerase Reverse Transcriptase (TERT). Indeed the expression of these two antigens is restricted to tumor cells, while being not expressed at all or to very negligible levels by healthy cells/tissues. Indeed, Survivin rescues cells from apoptosis (programmed cell death) and TERT promotes cell immortalization, two features, which are typical of de-regulated rather than healthy cells. Accordingly, Survivin is almost exclusively expressed during embryonic and fetal development but it becomes undetectable in terminally differentiated normal adult tissue, while it is re-expressed in tumor cell lines and several human cancer cells at a frequency of 34-100%. Also TERT protein, unlikely from its RNA subunit, is expressed almost only by tumor cells, even at very early stages of tumorigenesis. As a prognostic factor, Survivin and TERT expression is significantly associated with poor clinical outcome in cancers, such as breast cancer, ovarian cancer, neuroblastoma, colorectal cancer, lung cancer and esophageal cancer. As Survivin and TERT are preferentially expressed in tumor versus normal tissue, adverse effects on normal, differentiated cells are unlikely. Indeed, recent studies demonstrated that patient treated with DCs loaded with Survivin peptides or TERT mRNA or peptide do not develop any adverse events related to immune therapy, other than a local reaction at the vaccination site, despite induction of impressive immune reactivity against Survivin or TERT as measured by IFN-γ ELISPOT. Moreover, the strong immunological response against Survivin and TERT at the time of tumor response underscores the tumor regression, following vaccination.

Alternatively, the DCs of the present invention can be used to treat and/or prevent specific tumors. For example, in the case of breast cancer, DCs may be loaded with Her2-protein and Muc-1 mRNA.

Another possible combination of antigens (combination of a universal with a tumor-specific antigen) to be loaded on DCs is in the case of prostate cancer PSA-protein and Survivin-mRNA.

Finally, it is possible to load the DCs simultaneously with a mix of nucleic acid molecules and a mix of protein antigens.

The tumor associated antigens which are specifically expressed by specific tumor cells are known to the person skilled in the art. Therefore said person can choose those antigens which are required to produce loaded dendritic cells which can be used to treat or to prophylactically vaccinate individuals suffering from a distinct type of cancer or being at risk to get cancer.

The DCs of the present invention are thus loaded with two different types of molecules: nucleic acid molecule(s) and protein molecule(s).

Comparative studies suggest that nucleic acid, in particular mRNA, transfection is superior to other antigen-loading techniques in generating immunopotent DCs. Moreover, loading of DCs with, e.g., mRNA is simple and effective. The ability to amplify nucleic acid molecules from microscopic amounts of tumor tissue extends the use of DC vaccination to every cancer patient.

Loading of DCs with nucleic acid-encoding defined tumor antigens is simple, reproducible, and effective. Nucleic acid molecules corresponding to gene products, which sequence is known, can be rapidly generated *in vitro* using appropriate primers and polymerase chain reaction, in the case of mRNA reverse transcriptase-polymerase chain reaction (RT-PCR), coupled to transcription reactions. Manufacture of nucleic acids for clinical use of this *in vitro* generated non-cell-derived product can be performed in a cost-effective and defined manner, thus streamlining and simplifying the regulatory approval process. This contrasts with the complexities and limitations of using viral vectors, which limit their availability for investigational studies and restrict their clinical evaluation. Moreover, particularly the RNA approach offers a practical solution to a common problem encountered in this kind of therapy: the fact that in most cases, it is not possible to obtain sufficient tumor tissue to generate the amount of antigens needed for an effective and sustained immunization protocol for the cancer patient. Indeed, the RNA approach allows a simple and straightforward PCR-based amplification of the antigen of interest from microscopic amounts of tumor tissue, providing a virtually inexhaustible amount of antigen. It has been shown that incubation of immature human monocyte-derived DCs with antigen mRNA in medium alone is sufficient to sensitize the DCs to stimulate an antigen-specific CTL response, comparable if not slightly superior to that associated to DCs transfected in the presence of lipid.

As used herein the term "tumor associated antigen protein" refers to tumor associated antigens which comprise or consist of proteinaceous structures like polypeptides, proteins and peptides. Fragments of tumor associated antigen protein of the present invention have at least 10, preferably at least 20, more preferably at least 30, even more preferably at least 50, contiguous amino acid residues of said tumor associated antigens.

According to a preferred embodiment of the present invention the tumor associated antigen or fragment thereof encoded by the at least one nucleic acid molecule is selected from (a) the group of uTAAs, such as, but not restricted to Survivin and TElomerase Reverse Transcriptase (TERT) and/or (b) the group of tumor-specific Tumor Associated Antigens (TAAs), such as, but not restricted to PSA, muc-1 etc.. In a particularly preferred embodiment the tumor associated antigen or fragment thereof encoded by the at least one nucleic acid molecule is selected from the group consisting of Survivin and Telomerase Reverse Transcriptase (TERT).

The dendritic cells of the present invention have several advantages over loaded dendritic cells known in the art: (a) the simultaneous use of more antigens allows to increase the targeting range, even if the primary tumor shows a different (u)TAAs pattern of expression from secondary metastases and/or when the tumor progression is accompanied by changing in the (u)TAAs pattern of expression in one or more tumor focuses; (b)the use of uTAAs, possibly in combination with TAAs extends the applicability of the therapy to virtually any tumors; (c) the use of uTAAs, which expression is almost completely restricted to the tumor cells while increasing the efficacy of the therapy, drastically reduces the rising of any (severe) adverse effects; (d) the use of at least one (u)TAA encoded by a nucleic acid molecule and of at least one protein (u)TAA triggers and sustains the establishment of both the short term, transient cytotoxic response and the long-term, stable, adaptive response.

In particular, the simultaneous use of nucleic acid (e.g. RNA) and protein loading avoids the risk to trigger the T-reg response, which may be elicited by the only activation of the MHC-II through the processing and presentation of an exogenous protein antigens, while triggering two different responses: (a) the cytotoxic, fast and short-term response mediated by the CD8⁺ T-cells that recognize antigens coupled to MHC-I molecules. These cells directly attack other cells carrying the DC-presenting antigens on their surfaces. This kind of response is tightly controlled and generally requires a very strong MHC/antigen activation signal, and/or additional activation signals provided by "helper" T cells; (b) the memory, stable and long-term response mediated by the CD4⁺ T-cells (Th), which stimulate the activity of macrophages, CTL and B-cells, the latter producing antibodies. Indeed, T-helpers regulate both the innate and adaptive immune responses and help determine which types of immune responses the body will produce. Long-term active memory is acquired only by specific activation of B and T cells and requires that the antigens triggering such activation are strongly immunogenic.

The DCs of the present invention, loaded with at least one (u)TAA encoded by nuclear acid molecule and at least one (u)TAA protein antigen (a) mainly loose the capability to phagocyte. This feature is important to ensure the specific activation of the T-cells exclusively against the loaded antigens; (b) remain capable to migrate in response to stimuli, such as CCL19 and/or CD40L. This feature allows the DCs to move from the injection site towards the lymphonodes and/or where there is a high concentration of T-cells; (c) show a mature phenotype i.e. high surface expression of CD80, CD86, CD40, CD83, HLA-ABC and HLA-DR. This feature is important to dock and activate the right population of T-cells; (d) secrete mild to high levels of IL-12 and mild to none IL-10. This feature guarantees that the DCs sustain the cytotoxic response (IL-12) and potentially increase the activation of the T-helper cells (IL-10); (e) show the surface expression of the loaded antigen(s)upon stimulation with CD40L. This feature is important to determine the specificity of the DC stimulation of the T-cells; (f) upon harvesting and reculturing after a cycle of freezing/thawing show the typical dendritic morphology, characterized by adherence and presence of several fingers, elongating from the small, round cell body as well as by the presence of cell clusters. This feature constitutes a hallmark of DC functionality as antigen presenting cells (APCs; (g) can be stored between -80 and -180°C without loosing their active phenotype and/or functionality.

The present invention further relates also to the use of mature DCs, loaded with TERT and/or Survivin mRNA in combination with a protein tumor antigen or fragments thereof, preferably capable to migrate but not to phagocyte, in the treatment of patients, suffering from different solid and blood cancers.

The present invention, especially its preferred embodiments, offers numerous advantages over the prior art, such as the isolation of high quantity of pure monocytes from patient's PBMNCs, transformation rate of peripheral monocytes (pMos) into mature DCs between 40-80%, GMP- and re-vivo use conform protocols, standard production in GMP conform lab, the possibility of a serum-free process, a therapy virtually applicable to any type of solid and blood tumor, availability of high quantity of phenotypic and functional mature DCs, a ready-to-use product that can be stored for years, a strongly increased cytotoxic response, a strongly increased adaptive response, the inhibition of the T-reg response, a technique with minimal cell manipulation, no requirement for tumor tissue/cells, the possibility to expose tumor antigens with MHC-I as result of the mRNA loading coupled to simultaneous activation of the MHC-II as result of its combination with the antigen administered under the shape of protein, the combination of universal expressed and cancer-specific antigen, a high cell viability after a cycle of freezing/thawing, and the total preservation of cell functionality and phenotype after a cycle of freezing/thawing.

As used herein, the term "fragment" relates to a part or stretch of universal or type-specific tumor associated antigen, which retains at least one epitope of the *wild-type* protein. Consequently, a "fragment" of universal or type-specific tumor associated antigen, used to load dendritic cells, binds to at least one T-cell receptor, which promotes an immune response highly specific against cells expressing such universal or type-specific tumor associated antigen. The "fragment" relates as well to a part or stretch of universal or type-specific tumor associated antigen, modified and synthetized *in vitro* in order to increase its immunogenic activity.

The term "fragment" relates to both RNA and protein antigen.

The definition of "protein tumor antigen" includes as well tumor exudate and lysate in addition to entire and part of proteins and peptides, isolated or chemically/recombinantly produced. According to the present invention also tumor associated antigens and fragments thereof, which include amino acid modifications (deletions, substitutions, insertions) are included in the definition of tumor associated antigens and fragments thereof as used herein. This under the provision that these modified molecules still exhibit similar or even identical functionalities to the wild-type tumor associated antigen or fragment thereof.

According to a preferred embodiment of the present invention the nucleic acid molecule is a ribonucleic acid, preferably mRNA.

DCs loaded at the same time with mRNA and tumor-specific proteins/peptides almost completely loose the capability to phagocyte but retain the capability to migrate in response to stimuli; display high IL-12 production with or without mild IL-10 secretion; enhance T-cell cytotoxic response, since the antigen encoded by mRNA is processed as endogenous protein by DCs and therefore complexed with MHC-I; induce T-mem response through MHC-II, due to the fact that exogenous proteins are processed by DCs to be complexed with MHC-II; do not activate the T-reg.

The DCs are loaded with mRNA-encoding antigen for the following reasons:
- mRNA-transfected DCs are equally or more potent than peptide-pulsed DCs in stimulating CTL responses *in vitro* (Boczkowski, D. et al. (2000). Cancer Res 60(4): 1028-34);
- mRNA-transfected DCs are most effective APCs, capable of stimulating CD8+ responses *in vitro* (Gilboa, E. and J. Vieweg (2004). Immunol Rev 199: 251-63);
- mRNA transfection is so effective that it can induce and contribute to the DC maturation process ( Weissman, D. et al. (2000). J Immunol 165 (8): 4710-7 and Heiser, A. et al. (2002). J Clin Invest 109(3): 409-17);
- mRNA can be loaded on the DCs via passive transfection with high efficiency, as measured as CTL response (Nair, S. K. (1998). Gene Ther 5(11): 1445-6).

Although the mRNA and nucleic acid molecule loading method shows several advantages on other loading techniques, this approach has as well at least an Achilles' heel: although it triggers a potent CTL response through the activation of CD8⁺ cells, it cannot induce an effective CD4⁺ response, even if such a response has been shown critical for the establishment of an efficient immune response against tumor. Indeed, CD4⁺ cells provide important functions for the expansion and persistence of CD8⁺ CTLs; stimulate the innate arm of the immune system at the tumor site and inhibit local angiogenesis. An optimal anti-tumor immune response therefore requires the concomitant activation of both the CD8' and the CD4' T-cell driven immune response.

In order to achieve this goal, a second loading step, along with mRNA loading approach has been introduced: protein antigen loading by passive incubation. DCs stimulated by mRNA incubation, show an increased capability, for example, to phagocyte short peptides. Therefore, it turned out that the use of the double-loading approach in order to induce both CD8⁺ and CD4⁺ T-cell arms of the immune response is advantageous. This approach constitutes a great improvement of the DCs-based cancer immune therapy, since it can, at the same time, induce the short-time response by activation of the CD8⁺ as well as the long-lasting memory response, through the activation of the CD4⁺ T-cell.

The double loading approach has several advantages on other methods, known in the art:
● Specific activation of both the cytotoxic and the memory arms of the immune system;
● Absence of the T-reg response;
● Use of different antigens to direct the activation of each of the immune system arm with consequent gain in both sensitivity and strength of the stimulus;
● Therapy virtually applicable to any type of solid and blood tumor;
● Combination of universal and type-specific tumor antigens;
● Total conformity to GMP- and re-vivo use;
● Ready-to-use product that can be stored for years;
● Minimal cell manipulation;
● No requirement for tumor tissue/cells;
● Simultaneous activation of the MHC-I and the MHC-II complexes upon loading with (u)TAA(s) encoded by nucleic acid molecule(s)and (u)TAA(s)protein antigen(s), respectively;
● High cell viability after a cycle of freezing/thawing;
● High preservation of cell functionality and phenotype after a cycle of freezing/thawing;
● Versatility of the method.

According to another preferred embodiment of the present invention the nucleic acid molecule further comprises full-length, *wild-type* mRNA, a fragment of the *wild-type* mRNA, full-length, *in vitro* modified mRNA, a fragment of the *in vitro* modified mRNA, a mix of two or more full-length, fragments, *wild-type* or modified mRNAs.

According to a preferred embodiment of the present invention, the protein antigen comprises full-length, *wild-type* antigen; a fragment of the *wild-type* antigen; full-length, *in vitro* modified antigen; a fragment of the *in vitro* modified antigen; a mix of two or more full-length, fragments, *wild-type* or modified antigen; full-length or fragments of the antigen coupled with xenoantigens, such as but not only Keyhole Limpet Hemocyanin (KLH); tumor lysate and peptides, isolated or chemically/recombinantly produced. The term protein antigen comprises as well mix of antigen protein, peptide and lysate.

The dendritic cell production of the present invention may be loaded with any kind of tumor associated antigen. However, the tumor associated antigen is preferably a specific tumor associated antigen selected from the group consisting of carcinoembryonic antigen (CEA), mucin (MUC), prostate-specific membrane antigen (PSMA), BRCA and ras, and/or a universal tumor associated antigen selected from the group consisting of Survivin and TERT.

According to a preferred embodiment of the present invention the dendritic cells are of human or animal origin.

The dendritic cells of the present invention are preferably based on the expansion of autologous DCs from a human individual's peripheral blood. Peripheral Blood MonoNuclear Cells (PBMNCs) are collected through leukapheresis followed by elutriation or gradient centrifugation (i.e. Ficoll gradient centrifugation), in order to increase the monocyte (Mo) fraction, which constitute the selected DC precursors. This way to obtain Mos from individuals ensures both high purity and large amounts of DCs precursors that can be therefore cultured immediately, without the need of intermediate steps, such enrichment through the use of beads, which are on the one hand, not GMP-conformed and on the other hand, may be a source of contamination. The Mos are thereafter differentiated into DCs in a GMP-conform laboratory, with a culture medium, not only GMP-, but as well serum-free and re-vivo conformed.

The DCs are preferably loaded with the nucleic acid molecule(s) when they are still immature, matured and thereafter loaded with the protein antigen(s). Alternatively the DCs may be loaded with the nucleic acid molecule(s) first and subsequently with the protein antigen(s) when they are still immature and matured thereafter. In addition, the DCs may be loaded with the nucleic acid molecule(s) first and subsequently matured and loaded with the protein antigen(s) at the same time. Finally, the loading sequence nucleic acid molecule(s) first and protein antigen(s) after may be inverted. According to the method used, it is possible to modulate the immune response driven by the loaded DCs.

The DCs can be matured by means of adding one of the presently available standard cocktails, but preferentially by the use of Ribomunyl and INF-γ.

The mature DCs are preferably injected into the patients only if at least 50%, preferably at least 60%, more preferably at least 80% of them result to be double loaded.

Another aspect of the present invention relates to a method for manufacturing dendritic cells loaded with at least one nucleic acid molecule encoding a tumor associated antigen or fragment thereof and at least one protein molecule being a tumor associated antigen or fragment thereof, preferably according to the present invention, comprising the steps of:
● Providing dendritic cells;
● Loading said cell with at least one nucleic acid molecule encoding a tumor associated antigen and at least one protein molecule being a tumor associated antigen or fragment thereof as described above; and optionally
● Maturing said DCs.

According to a preferred embodiment of the present invention the dendritic cells source consists of their autologous precursors, i.e. PBMNC-derived monocytes. The monocytes are transdifferentiated into immature DCs upon 5-6 days culture in CellGro, added up with GM-CSF and IL-4.

The dendritic cells may be alternatively directly isolated from whole blood. Protocols for direct isolation of dendritic cells and transdifferentiation of monocytes into dendritic cells are known in the art. The present protocol ensures that 40-80% of the initial Mos become DCs, whereas current protocols guarantee about 10-20%.

The DCs are finally matured *in vitro* in order to increase the levels of the costimulatory molecules and to trigger a strong and durable tumor-specific T-cell cytotoxic response. The maturation-cocktail according to the present invention is completely GMP-conform and ensures a prolonged IL-12 production, without or with mild IL-10 secretion in comparison with the currently employed methods. Moreover, these DCs are able to produce high levels of IL-12 and mild amounts of IL-10 upon stimulation with CD40L and remain viable, phenotypic, morphological and functional active upon a freezing/thawing cycle even after one year of deep-frozen preservation in DMSO 10% or Glycerol 10% (-150°C). When the method described in this patent is used to manufacture a GMP-conformed final product, its releasing is conditional to the successful pass of a strict End Product Control (EPC), designed to determine soundly the viability, the phenotypic, morphological and functional activity, the stability upon deep-frozen preservation and a freezing/thawing cycle, the purity and the sterility of the DCs, which must be applied to the patient. The final product is preferentially stored in glycerol (10%) or DMSO (10%), i.e. freezing media that make the product ready-to-use by preserving its quality for a long period, that are GMP-conformed and that do not trigger side effects at the used concentrations. These freezing media grant as well the long-lasting activity (IL-12 secretion, T-cells stimulation and migration) of the product. Double loaded DCs loose almost completely their capability to phagocyte but retain their capability to migrate in response to stimuli and to a very lower degree, to migrate spontaneously, display high IL-12 production with or without mild IL-10 secretion; and enhance T-cell cytotoxic and T-mem response, without triggering the activation of the T-reg.

According to another preferred embodiment of the present invention the immature dendritic cells are matured upon or after loading as well as during the protein antigen loading.

The dendritic cells are preferably matured by adding Ribomunyl and INF-γ.

Another aspect of the present invention relates to dendritic cells transdifferentiated and/or cultured, loaded and matured according to the method described in the present invention.

Yet another aspect of the present invention relates to a pharmaceutical/clinical grade preparation comprising dendritic cells according to the present invention, since the dendritic cells of the present invention may be provided in a pharmaceutical/clinical grade preparation. Said preparation may be employed in the treatment or prevention of virtually any type of solid and blood cancers. The double loaded, mature DCs are preferentially inoculated weekly or fortnightly. Since this product consists of an autologous product and therefore may show many individualistic components, the route of administration may vary between weekly to monthly administration, according to the results observed in the patient during the continuous monitoring. The DCs can be administered preferentially intradermally or intranodally. Alternatively they can be administered para-, peri-nodally, subcutaneously or intravenously, depending on the cancer treated.

The DCs are preferably administered as close as possible to the tumor. The standard dose comprises preferably approximately 7-13*10⁶ DCs, solved in a final volume of 0.5-1 mL of freezing medium.

The therapy is made up of a number of injections comprised between 2 and 20. The product is preferentially but not exclusively stored in 10% glycerol or 10% DMSO thawed for a max of 10' at RT and immediately administered.

According to a preferred embodiment of the present invention the administration of the product can be accompanied by the administration of immunostimulatory agents and/or adjuvants. Its usage is as well suitable in concomitance with chemotherapy as well as during the pauses between chemotherapic cycles but not in concomitance with immune suppressive treatments and/or in presence of severely reduced amounts of T-cells (preferential targets of the DC-driven activation of the immune system against cancer).

A further aspect of the present invention relates to the use of dendritic cells according to the present invention for the manufacture of a vaccine for treating or preventing cancer in an individual.

The dendritic cells of the present invention, which are loaded with at least one nucleic acid molecule encoding for a tumor associated antigen (see above) and with at least one protein molecule being a tumor associated antigen (see above) may be suitable used to treat an individual suffering from cancer or to prevent that an individual develops cancer.

The present invention is further illustrated by the following figures and example, however, without being restricted thereto.
Fig. 1 shows the expression of Survivin and PSA in immature, semi-mature, unloaded DCs and mature, Survivin mRNA- as well as Survivin mRNA + peptide loaded DCs, cultured as described in the examples.
Fig. 2 shows the phenotype of immature, semi-mature, unloaded DCs and mature, Survivin mRNA- as well as Survivin mRNA + peptide loaded DCs, cultured as described in the examples.
Fig. 3 shows the secretion of IL-10 and IL-12 by immature, semi-mature, unloaded DCs and mature, Survivin mRNA- as well as Survivin mRNA + peptide loaded DCs, cultured as described in the examples.
Fig. 4 shows the induction of T-cell proliferation upon incubation with immature, semi-mature, unloaded DCs and mature, Survivin mRNA- as well as Survivin mRNA + peptide loaded DCs, cultured as described in the examples.
Fig. 5 shows the spontaneous and induced (by CCL19) cell migration displayed by immature, semi-mature, unloaded DCs and mature, Survivin mRNA- as well as Survivin mRNA + peptide loaded DCs, cultured as described in the examples.
Fig. 6 shows the spontaneous and induced (by CCL19) phagocytosis displayed by immature, semi-mature, unloaded DCs/mRNA loaded DCs and mature, Survivin mRNA-loaded DCs, cultured as described in the examples.
Fig. 7 shows the stimulation of T-cells cytotoxic and T-mem upon incubation with double-loaded DCs, whereas the T-reg number was strongly down-regulated upon incubation with double-loaded DCs.

### EXAMPLES:

### 1. Pre-clinical pilot experiments

Monocytes are isolated from patients' leukapheresate and monitored for the following markers:

| FITC | PE | ECD | PC5 | PC7 |
|---|---|---|---|---|
| CD14 | CD19 | CD3 | CD16 | CD45 |
| CD41 | GPA | n.a. | n.a. | CD45 |

Subsequently, PBMNCs collected by leukapheresis are ficolized, recounted and re-monitored as described before. At this point, Ficolized-cells or cells derived from elutriation are set for the adherence for 2h in CellGro, added up with GM-CSF (1000-2500 U/mL) and IL-4 (400-1000 U/mL). Afterwards, the non-adherent cells are discarded with the supernatant and the adherent cells are further cultured in CellGro, added up with GM-CSF (1000-2500 U/mL) and IL-4 (400-1000 U/mL), in a concentration of 1-2 Mio/mL for 5-6 days in order to transdifferentiate the monocytes into immature DCs. Thereafter, the cells are loaded with mRNA. Shortly, DCs are washed twice in PBS, counted, and spun at 300xg for 10'. They are resuspended in CellGro medium and incubated in mRNA-containing solution (0.1-100 µg RNA/1x10⁶ DCs) for 2-4h in a humidified incubator at 37°C/5% CO₂. Without washing, the cultures are subsequently incubated with the protein antigen solution (0.1-100 µg peptide/1x10⁶ DCs) for 2-12h in a humidified incubator at 37°C/5% CO₂. Thereafter, without washing, the cultures are added up with Ribomunyl (0.1-10 µg/mL) and INF-γ (400-1000 U/mL) and let mature for 8-24h. The matured DCs are stored in aliquots of at least 10 millions DCs each in Glycerol(aqueous solution) 10%.

The quality control (QC) is performed as described below: one aliquot of cells is thawed and put in culture for 48h.

### Phenotype:

| FITC | PE | PC5 | PC7 |
|---|---|---|---|
| CD80 | CXCR4 | CD83 | CD45 |
| CD80 | CD19 | CD3 | CD45 |
| HLA-ABC | HLA-DR | CD40 | CD45 |

Mature DCs show the up-regulation of CXCR4, CD40, CD80, CD83, HLA-ABC and HLA-DR.

Cell viability is measured by Casy Counter with a GMP-conform software and must be at least ≥ 70%.

The contamination by T-cells and B-cells is monitored by detecting via FACS the number of CD3 and CD19 positive cells. The contamination is always kept under 25%.

### Functionality:

The immune activity test is performed through the analysis of the production of IL-12 vs IL-10 through an ISO 9002 validated ELISA assay and induction of lymphocytes proliferation via mixed leukocyte reaction.

The phagocytosis and migration activity of the dl-DCs is monitored by FITC-dextran uptaking and Transwell-assay, respectively.

### 2. Clinical study design

*Title:* "A Phase I, Open, Randomized Study to Investigate the Safety of Active Immunotherapy with Fully Mature, TERT-mRNA and Survivin-peptide Double Loaded DCs in Subjects with Advanced Epithelial Ovarian Cancer, 8 Weeks After Completed Adjuvant Therapy"

### Phase: Phase I

### Indications: Advanced epithelial ovarian cancer

*Study objectives:* To investigate the safety of active immune therapy with fully mature, TERT (Telomerase Reverse Transcriptase)-mRNA and Survivin-peptide double loaded DCs (Dendritic Cells) in patients with advanced ovarian cancer, 8 weeks after completed adjuvant therapy. Efficacy in terms of the reduction of Disseminated Tumor Cells (DTC) will be investigated as a secondary objective.

*Study design:* This is an open phase I trial in patients with advanced epithelial ovarian cancer. Patients will be randomized into the treatment arm A with weekly administration vs. treatment arm B with bi-weekly administration. Each patient will receive a total of 8 intradermal injections. The treatment course comprises of 1 injection once a week for 8 times for treatment group A and of 1 injection once in a fortnight for 8 times for treatment group B.

Patients will be followed for safety parameters and the tumor status will be assessed at weeks 12, 24, 36, 48, 72, and 96 until disease progression. After the end of treatment (week 7 for treatment arm A and week 14 for treatment arm B), patients will be followed for 24 months until disease progression, death, or end of study. After disease progression, patients will be followed until death or end of study.

In order to comply with the cGCP guideline rules, the first 6 patients will be observed under controlled conditions for 48 hours. They will be treated sequentially, at intervals of 48 hours.

### Planned sample size: 25 patients.

*Treatment duration:* Each patient will receive a total of 8 intradermal injections, administered paraumbilically. The treatment course comprises of 8 injection cycles (1 injection per cycle), once a week for treatment arm A and once in a fortnight for treatment arm B.

*Study treatment:* The IMP consists of 1*10⁷±30% autologous, fully mature DCs, double loaded with TERT-mRNA and Survivin-peptide, diluted in 0.5 mL of a standard freezing solution, made up of 10% DMSO, solved in a physiologic water solution of 5% glucose; contained in a 2 mL, GCP-conformed labeled cryovial. The IMP is supplied deep-frozen and once thawed at RT for a maximum of 10 min, it must be immediately administered.

The production of the IMP is performed as described below:
The patients undergo leukapheresis and the collected PBMNCs are transported within 24 hours between 2-20°C to the clean room facility, where the monocyte fraction is enriched via Ficoll density gradient centrifugation. After Ficoll, the purity of the monocytes is increased by seeding the resultant cells in plain CellGro and incubating them for 2 hours at 37°C/5% CO₂. At this point, the non-adherent cells (lymphocytes) are washed out and the adherent ones (monocytes) are further cultured at a final concentration of 1-2*10⁶/mL in CellGro, supplemented with IL-4 and GM-CSF.

Once seeded in presence of IL-4 and GM-CSF, the cells are cultured for 5 days. At this point, they are washed, seeded in plain CellGro and incubated at first with TERT-mRNA for 2h. At this point, without washing step, the cells are fully matured by incubating them at 37°C/5% CO₂ in CellGro, supplemented with Ribomunyl and INF-γ for 16h. At this point the adherent, mature cells are washed once in PBS and finally added up with Survivin-peptide for 2h at 37°C/5% CO₂. Thereafter the cells are harvested, counted and tested for viability and purity, before being aliquoted into vials of 1*10⁷±30% cells each, and frozen in 0.5 mL of freezing solution, made up of 10% DMSO, solved in a physiologic water solution of 5% glucose.

The IMP harvesting includes the storage of 8 IMP vials to be injected into the patient; 1 sterility sample; 1 retain sample; 3-5 EPC samples and potential overage samples. The product release is strictly conditional to its compliance with the EPC criteria accordingly to sterility, viability, phenotype, purity, functionality and stability. Once that the batch has been officially released, each IMP vial is singularly delivered in dry ice to the study site, where it can be stored in the sealed transport box for a maximum of 24h. Once thawed at RT for a maximum of 10 min, the IMP must be immediately injected within 10 min intradermally and paraumbilically into the patient.

### 3. Proof of Principle

Monocytes isolated from healthy controls' leukapheresates undergo a subsequent enrichment through elutriation and are thereafter monitored for the following markers:

| FITC | PE | ECD | PC5 | PC7 |
|---|---|---|---|---|
| CD14 | CD19 | CD3 | CD16 | CD45 |
| CD41 | GPA | n.a. | n.a. | CD45 |

and counted. At this point the monocytes are divided in aliquots of 5*10⁷ cells each and frozen in DMSO 10%, diluted in an aqueous solution of 5% glucose to a final volume of 1.8 mL. The aliquots are stored at -150°C until use. At least 14 aliquots belonging to the same batch are thawed at the same time and pooled to perform each experiment. Upon thawing the cells are washed once in PBS,monitored for the said markers, viability and amount. Thereafter they are seed in CellGro, added up with GM-CSF (1000-2500 U/mL) and IL-4 (400-1000 U/mL). Afterwards, the non-adherent cells are discarded with the supernatant and the adherent cells are further cultured in CellGro, added up with GM-CSF (1000 U/mL) and IL-4 (400 U/mL), in a concentration of 1-2 Mio/mL for 5 days in order to transdifferentiate the monocytes into immature DCs. Thereafter, the cells are loaded with mRNA. Shortly, DCs are washed once in PBS, counted, and spun at 1000 rpm for 10'. They are resuspended in CellGro medium and incubated in mRNA-containing solution (50 ng RNA/1*10⁶ DCs) for 2h in a humidified incubator at 37°C/5% CO₂. Without washing, the cultures are subsequently added up with Ribomunyl (100 µg/mL) and INF-γ (1000 U/mL) and let mature for 16h. At this point the cells are washed once in PBS and reseeded in plain CellGro, added up with the protein antigen solution (1-10 µg peptide/1*10⁶ DCs) and incubated for 2h in a humidified incubator at 37°C/5% CO₂. Depending on the experimental set, the peptide was alternatively loaded before maturing or in contemporaneous with the maturing cocktail. Thereafter, the cells are washed once in PBS and the adherent ones are harvested and immediately processed or alternatively divided in aliquots of 1*10⁷ DCs in DMSO 10%, diluted in an aqueous solution of 5% glucose to a final volume of 0.5 mL.

The quality control (QC) is performed as described below.

Upon harvesting or upon a freezing/thawing cycle the cells are put in culture for 12-24h.

Upon starting of the QC and at specific time-points (0-4-12/14 and 24h) the cells are monitored for the following parameters:

| **FITC** | **PE** | **PC5** | **PC7** |
|---|---|---|---|
| CD14 (My4) | CD19 | CD3 | CD45 |
| CD80 | CD40 | CD83 | CD45 |
| Survivin | HLA-DR | HLA-ABC | CD45 |

| TERT | HLA-DR | HLA-ABC | CD45 |
|---|---|---|---|
| TERT | Survivin | CD83 | CD45 |

In addition to morphology, viability and secretion of IL-10 and IL-12.

### Materials and Methods

### 1. Pre-clinical pilot experiments

### 1.1 Cell isolation

Leukaphereses and elutriations are performed with GAMBRO equipment and following the manufacture instruction.

### 1.2 Ficoll purification

10 mL of blood/apheresate are mixed with 30 mL of PBS + 10% citrate buffer. Thereafter 30 mL of the mix are added to 20 mL of Ficoll and centrifuge 20' at 2000 rpm at RT. Afterwards the interfaces are collected and centrifuged 10' at 1200 rpm at RT. Finally, the pellets are resuspended in PBS, washed twice 10' at 1200 rpm at RT and resuspend in CellGro.

### 1.3 Cell culture

DCs are cultured into plastic flasks in CellGro added up with GM-CSF (400-1000 U/mL) and IL-4 (100-400 U/mL) 5-6 days and matured upon addition of Ribomunyl (0.5-1 µg/mL) and INF-γ (200-1000 U/mL) for 24h.

### 1.4. FACS

50 µL of whole blood or 50 µL of cell-suspension (thawed DCs, in a final concentration of 1 x 10⁶ cells/mL in PBS, 1%BSA, 0.1% NaN₃) are incubated 15' at room temperature with the appropriate amount of FACS antibodies, according to the manufacturer's recommendations (erythrocytes of whole blood are chemically lysed), washed at 800 rpm (RT) with 1.5 mL or 0.8 mL of PBS respectively, resuspended in 0.5 mL of PBS, supplemented with fixative and further processed by FACS®.

### 1.5 ELISA

Frozen DCs are thawed and re-cultured in RPMI supplemented with 2% HS at a final concentration of 1x10⁶ cells/mL. After 24h, the supernatant is harvested and analysed for human IL-10 ad IL-12p70, using CellScience ELISA KIT ISO 9002 and following the product instructions.

### 1.6 Mixed leukocyte reaction

Frozen DCs are thawed, and co-cultured at a final concentration of 1x10⁴ cells/well in a 96-well U-bottom plate with the non-adherent fraction of Ficoll-separated PBMNCs from a healthy donor at a final concentration of 1*10⁶ per cells/well in RPMI with 2% human serum (off the clott, AB), supplemented with 10% AlamarBlue® at a final volume of 150 µL. Proliferation causes reduction of the dye changing from oxidized (non-fluorescent, blue) form to reduced (fluorescent, red) form. For 2-7 days, every 4h, the absorbance is measured in a plate-reader at 595 nm with reference wavelength at 620 nm.

### 1.7. DTCs isolation

The DTCs isolation is performed according to the protocol provided with OncoQuick kit (Gentech).

### 1.8. Survivin mRNA transcription and purification

Survivin mRNA transcription and purification are performed, according to the manufacturer's protocols, through the use of mMESSAGE mMACHINE® T7/T3 Kits (Ambion) and of Oligotex mRNA Mini Kit (Qiagen), respectively.

### 1.9 Protein synthesis

All the used tumor specific proteins/peptides are synthesized under GMP conform rules by the American Peptide Company, Inc. or equally certified companies, which produce GMP-grade peptides for clinical use. Protein antigens are prepared starting from the relative cDNA(OriGene), according to the instruction and by means of the research grade "EasyXpress Protein Synthesis" Kit (Qiagen).

### 1.10 Western blot

The cells are lysed in Frackelton buffer for 20' in ice and centrifuged at 1000g 10' at 4°C. After collecting the supernatant, the protein concentration is measured with the Bradford assay and blue sample buffer is added to each sample in a ratio of 4:1. After preparing a polyacrilamide gel, the proteins are run and blotted on a nitrocellulose membrane. Thereafter, the membrane is washed in TBS-T and incubated at first 1h in about 10 mL of blocking solution, made up of TBS-T 1X added up with 0.5 mL of Tween-20 and milk 5% and then in 10 mL of the first antibody diluted in TBS-T w/o milk, added up of 3% BSA and 0.02% NaNₛ, o/n. Thereafter the membrane is washed thrice in TBS-T and incubated with the secondary antibody for 40' at RT. Finally the protein expression is detected with the ECL kit.

### 2. Clinical study and Proof of Principle

### 1.1 Cell isolation

Leukaphereses and elutriations are performed with GAMBRO equipment and following the manufacture instruction.

### 1.2 Monocyte freezing

The cells obtained by elutriation are counted and characterized by FACS. Finally the monocytes are divided in aliquots of 3*10⁷ cells each and frozen in DMSO 10%, diluted in an aqueous solution of 5% glucose to a final volume of 1.8 mL. The aliquots are stored at -150°C until use.

### 1.3 Cell culture

DCs are cultured into plastic flasks in CellGro added up with GM-CSF (1000 U/mL) and IL-4 (400 U/mL) 5 days and matured upon addition of Ribomunyl (100 µg/mL) and INF-γ (1000 U/mL) for 16h.

### 1.4. FACS

50 µL of cell-suspension (from a sample reaching a final concentration of 1*10⁶ cells/mL in PBS, with or without 0.5% BSA, 2mM EDTA) are incubated 15'in the refrigerator and in the dark with the appropriate amount of FACS antibodies, according to the manufacturer's recommendations, washed at about 4°C at 1000 rpm with 1.0 mL of PBS with or without 0.5% BSA, 2mM EDTA, resuspended in 0.5 mL of PBS, supplemented with fixative and further processed by FACS®.

### 1.5 ELISA

Harvested or frozen DCs are thawed and re-cultured in CellGro in presence or in absence of CD40L at a final concentration of 1*10⁶ cells/mL. At specific check-point, scattered over 24h, the supernatant is harvested and analysed for human IL-10 ad IL-12p70, using CellScience ELISA KIT ISO 9002 and following the product instructions.

### 1.6 TERT mRNA transcription and purification

TERT mRNA transcription is performed, according to the manufacturer's protocols, through the use of mMESSAGE mMACHINE® T7/T3 Kits (Ambion).

### 1.7 Survivin Peptide

Survivin peptide has been delivered by Global peptide or American Peptide Company, Inc. as liophylized product. Once reconstituted in water, it has been stored at -80°C in aliquots equal to 100 µg/mL each.

### Results

### ● Pre-clinical pilot experiments

All the results showed in this section were performed on DCs, cultured as described previously and frozen in Glycerol 10%. Cell viability, after thawing, was >75%. The results obtained with 10% Glycerol solution are comparable or slightly superior to those showed upon freezing with DMSO 10%. Freezing cells in glycerol has the major advantage on freezing them with DMSO that it does not require a centrifuging step to eliminate toxic substances (DMSO) and therefore it allows an easier way to inject the DCs into the patients, without special tools and equipment (centrifuge, lamina flow, steril environment) requirement.

To grant that DCs incubated with Survivin mRNA and protein antigen (PSA) were indeed loaded, a Western Blot to determine whether the phagocyted mRNA was as well translated and whether the protein antigen was expressed by the DCs was performed.

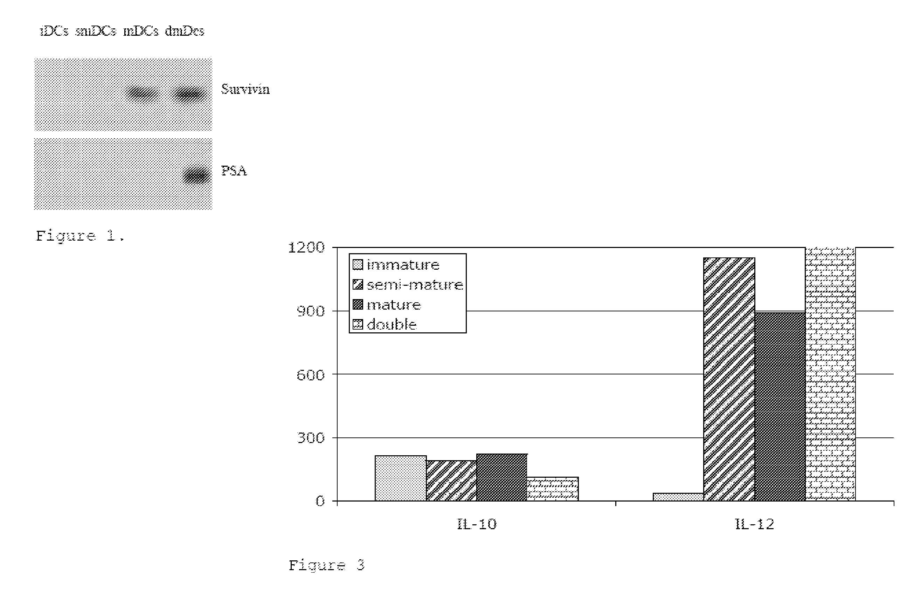

1 Mio cells were washed twice in PBS, lysed in Frackelton buffer and processed by Western Blot. The results reported in Fig. 1 show that immature DCs (iDCs) and unloaded, semi-mature DCs (smDCs) do not express neither Survivin nor PSA. Mature DCs, loaded with Survivin mRNA (mDCs), shows the expression of this antigen but not of PSA and mature DCs, loaded with Survivin and PSA (dmDCs), show the expression of both antigens. These results confirm that the loading procedure is functional for both mRNA and protein loading and it is very efficient, without the need of carriers or active transfection means.

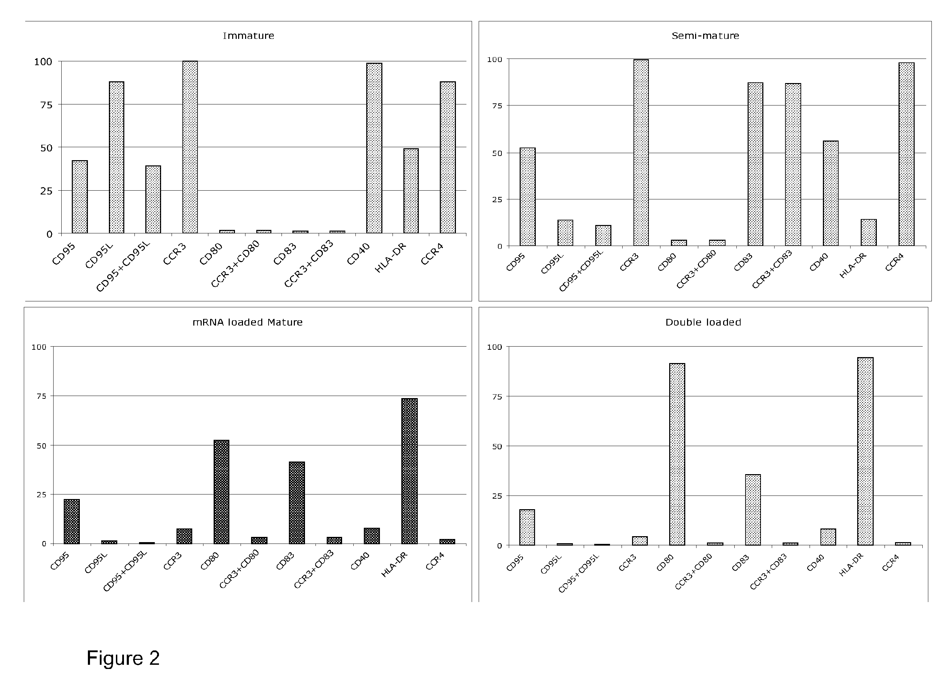

In 10 patients it has been shown that DCs, cultured as described in the present protocol expressed different CDs groups on their surface, according to the different phases of maturation. Not stimulated DCs do not express maturing markers such as CD80 and CD83, but expressed immature markers, such as CCR3. Semi-mature, unloaded DCs show a combination of immature markers, such as CCR3 as well as low CD80, and of mature markers, such as CD83. Fully mature DCs, loaded with Survivin mRNA showed only mature markers, such as CD80 and CD83, but no immature markers, such as CCR3 (Fig. 2).

To determine whether the present protocol allows not only the phenotypic but as well the functional maturation of the DCs, the secretion of IL-10 and IL-12 in the supernatant was analysed. IL-12 is known to enhance the ability of the immune system to kill tumor cells and may interfere with blood flow to the tumor. IL-12 enhances tumor clearance by stimulating the activation of the cytotoxic T-cell. On the other side, IL-10 has been shown to have a significant inhibitory effect on several aspects of DC function; such as the expression of costimulatory molecules and the ability to synthesize IL-12. Importantly, IL-10-treated DCs become tolerogenic. Normally, iDCs do not produce neither IL-10 (or very slightly) nor IL-12, whereas mDCs secrete high levels of IL-12 and very low if any levels of IL-10.

As expected, iDCs did not produce IL-12 and only a minimal quantity of IL-10, whereas semi-mature, unloaded DCs, Survivin mRNA loaded, mature DCs and Survivin mRNA + protein loaded, mature DCs cultured following our protocol produce high levels of IL-12 and very low levels of IL-10 (Fig. 3).

To further demonstrate that the DCs according to the present invention are functional active, their effects on heterologous T-cell proliferation through a mix-leukocyte reaction is assessed.

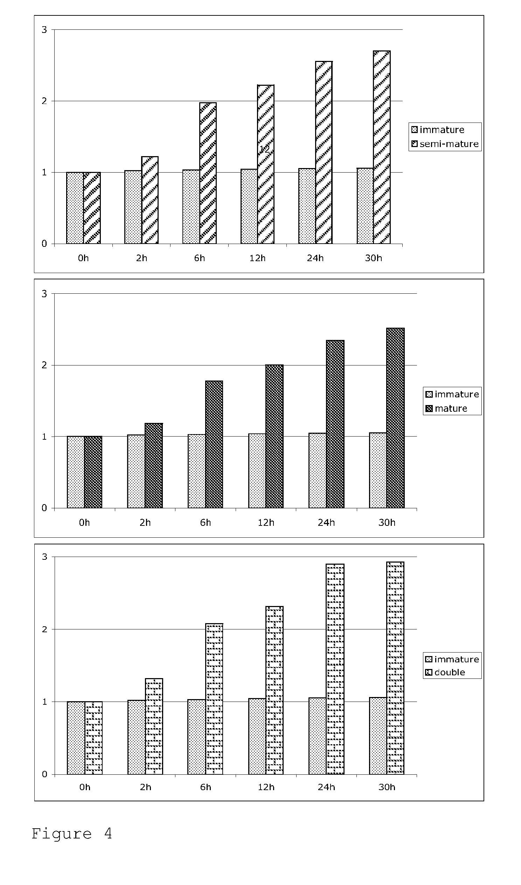

Incubation of semi-mature, unloaded and mature, Survivin mRNA/Survivin mRNA + protein loaded DCs increased proliferation of T-cells, showing that DCs cultured as described in the present example display a high positive effect on at least one of the major players of the immune system (T-cells), showing a potential benefit on tumor clearence. As expected, incubation of iDCs with T-cells did not increase their proliferation rate (Fig. 4).

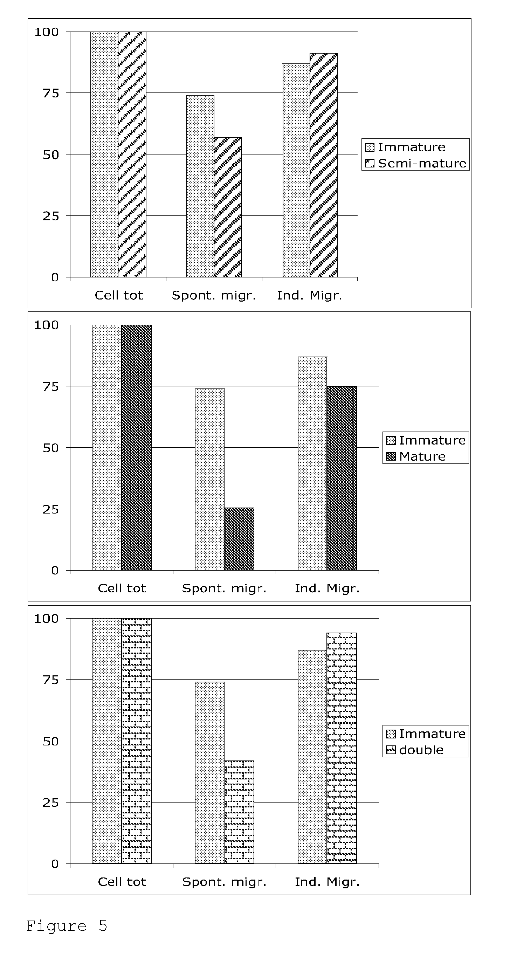

iDCs are characterized by a strong migration potentiality, which is not inducible by CCL19 addition due to lack of CXCR4/CCR7 expression on iDCs. Semi-mature DCs, on the contrary, respond to CCL19 induction but still retain spontaneous migration ability, even if to less extend, compared to iDCs. Mature, loaded DCs (almost) completely lose the capability to migrate spontaneously but they do migrate upon CCL19 induction (Fig. 5). This means that semi-mature unloaded DCs as well as mature, Survivin mRNA + protein-loaded DCs are able to move from the injection site towards the stimulus source (i.e. tumor site) and therefore they need not be injected directly intranodally, but they can as well be injected in the surrounding of the tumor. This fact makes easier the use of the DC vaccine, since it does not require specific tools, such as ultrasound device, to determine exactly the lymphonode position.

Finally it was investigated whether DCs cultured as described in the protocol were still able to actively phagocyte spontaneously or in response to CCL19.

iDCs are characterized by a strong phagocytotic capability, which is not inducible by CCL19, due to lack of CXCR4/CCR7 expression on iDCs. Semi-mature DCs and Survivin mRNA-loaded DCs, on the contrary, respond to CCL19 induction but still retain spontaneous phagocytosis ability, even if to less extend compared to iDCs. Mature, Survivin mRNA and protein loaded DCs completely loose the capability to phagocyte both spontaneously and upon CCL19 induction.

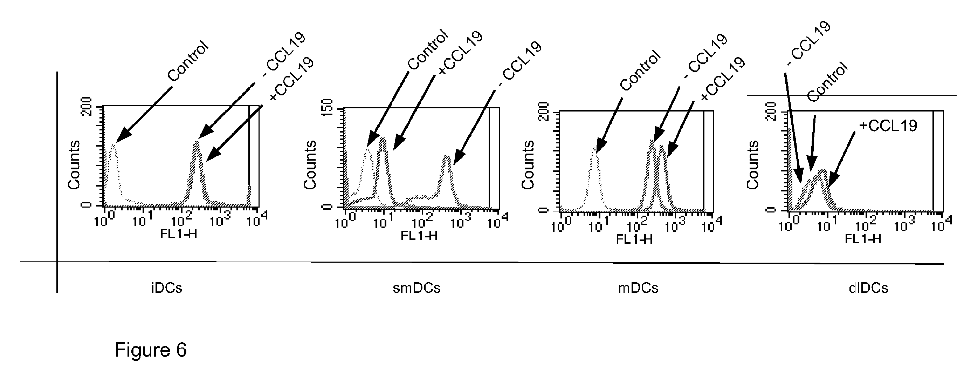

This means that semi-mature, unloaded DCs as well as mRNA loaded DCs, prepared following the protocol of the present invention are ready to phagocyte tumor antigen, showing a very active phenotype that may strongly increase the direct immune response to the tumor, while double loaded (mRNA + protein) and fully mature DCs are ready to induce T-cell proliferation and activation but loose their ability to further phagocyte tumor tissue, increasing the specificity of the immune response towards the already uptaken antigen (Fig. 6).

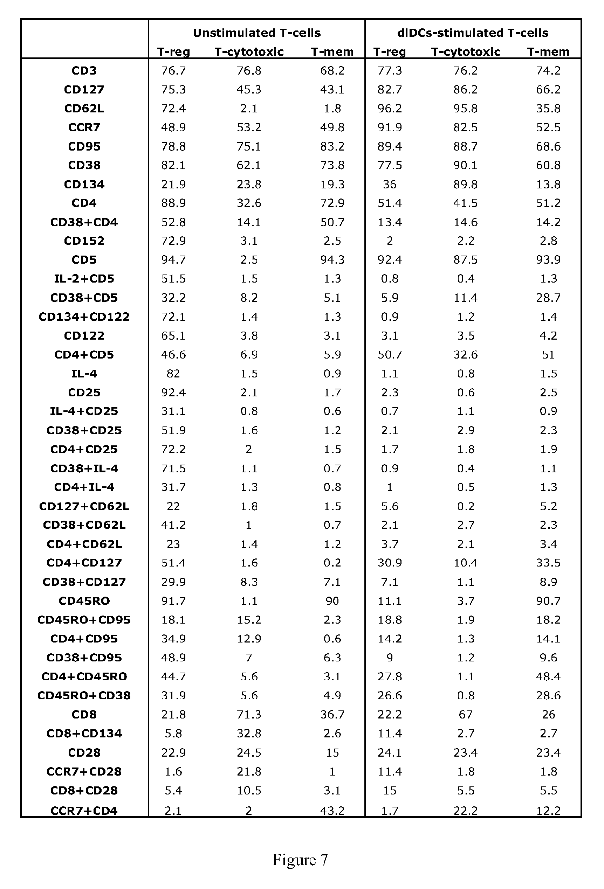

Finally, the double loaded DCs, with mRNA and protein, were shown able to induce a high cytotoxic and memory response without triggering the activation of T-reg response, upon *in vitro* incubation with allogenic lymphocytes (Fig. 7).

### 2. Proof of Principle

All the results showed in this section were performed starting from DCs, cultured from frozen monocytes derived from elutriations of healthy controls. The following batches were compared:
● **Batch 1:** the monocytes were transdifferentiated for 5 days into immature DCs (iDCs), loaded with TERT mRNA, matured and finally loaded with Survivin. The cells were not frozen but immediately analysed at harvesting and upon 24h reculturing at time points: 0h (harvesting); 4h and 24h;
● **Batch 2:** the monocytes were transdifferentiated for 5 days into immature DCs (iDCs), loaded with TERT mRNA, matured and loaded with Survivin at the same time. The cells were frozen in DMSO and analysed at harvesting, upon thawing and after 4 and 24h reculturing;
● **Batch 3:** the monocytes were transdifferentiated for 6 days into immature DCs (iDCs), loaded with TERT mRNA, matured and finally loaded with Survivin. The cells were frozen in DMSO and analysed upon thawing and after 12 and 24h reculturing.
The monocyte viability, upon thawing, was always ≥ 70%. During the proof of principle, different loading methods were compared:
● **Batch 1:** 2h incubation with 50 ng/mL TERT-mRNA, followed by 16h maturation and subsequent 2h incubation with 10 µg/mL Survivin-peptide;
● **Batch 2:**2h incubation with 50 ng/mL TERT-mRNA, followed by 16h incubation with 10 µg/mL Survivin-peptide and maturation cocktail, administered at the same time;
● **Batch 3:** 2h incubation with 50 ng/mL TERT-mRNA, followed by 16h maturation and subsequent 2h incubation with 10 µg/mL Survivin-peptide.

Upon 5 days of culture in CellGro added up with GM-CSF and IL-4, more than 50% of the initial amount of monocytes became immature DCs, half of them showing an adherent phenotype. On the contrary, if the cells were cultured for 6 days, only 10% of the cells were adherent.

After 2h incubation in presence of TERT-mRNA at least 70% of the cells became adherent. When Survivin-peptide was administered with the maturing cocktail, the cells formed nice clusters, while if Survivin peptide was administered after the maturing cocktail, the cells tended to show longer dendrites and to avoid the formation of tight clusters.

After completion of loading (in any form) and maturation, the cells were more than 95% adherent. In case the cells were cultured in presence of GM-CSF and IL-4 for 6 instead of 5 days, the adherent cells constituted about the 50% of the entire amount.

At harvesting, cell viability was comparable among the 3 batches, showing that time of transdifferentiation, loading method and maturing schedule did not affect this parameter, which remained always above the limit of 70% (Nicolette et al., 2007).

Upon a freezing/thawing cycle, cell viability was comparable between the 2 batches analysed and only slightly decreased if ever in comparison with the results obtained at the harvesting point. Again, loading method and maturing schedule did not affect viability, which remained always above the limit of 70%:
The viability of batches cultured according to different variations of the main protocol is comparable upon harvesting and upon thawing.

| **Batch** | **Reference in %** | **At harvesting** | **Upon Thawing** |
|---|---|---|---|
| 01 | ≥ 70 | 80.2 | n.a. |
| 02 | ≥ 70 | 84.4 | 77.7 |
| 03 | ≥ 70 | 78.8 | 78.4 |

The loaded antigens TERT and Survivin were never detectable on cell surface upon harvesting or just upon thawing by FACS. Upon reculturing, the surface expression of the two antigens increased between 4 and 12h. From 12 to 24h it remained stable. If the cells were stimulated with CD40L, after 4h reculturing, the surface expression of the 2 markers increased in comparison with that detected on cells cultured in plain medium. Nevertheless, after 12h, when the expression of the 2 antigens reached its plateau, the effects of CD40L became undetectable:
**a)** Detection of the loaded antigens (TERT and Survivin) between harvesting and 24h reculturing in plain medium;

| **TERT and Survivin expression on cell surface in %: plain medium** | | | | | |
|---|---|---|---|---|---|
| **Batch** | **At harvesting** | **Upon Thawing** | **Upon 4h Reculturing** | **Upon 12h Reculturing** | **Upon 24h Reculturing** |
| 01 | n.d. | n.a. | 30.2 | n.a. | 82.5 |
| 02 | n.d. | n.d. | 27.6 | n.a. | 96 |
| 03 | n.d. | n.d. | n.a. | 90.8 | 91.5 |

**b)** Detection of the loaded antigens (TERT and Survivin) between harvesting and 24h reculturing in presence of CD40L stimulation.

| **TERT and Survivin expression on cell surface in %: in presence of CD40L stimulation** | | | | | |
|---|---|---|---|---|---|
| **Batch** | **At harvesting** | **Upon Thawing** | **Upon 4h Reculturing** | **Upon 12h Reculturing** | **Upon 24h Reculturing** |
| 01 | n.d. | n.a. | 47.4 | n.a. | 84.9 |
| 02 | n.d. | n.d. | 35.2 | n.a. | 95.2 |
| 03 | n.d. | n.d. | n.a. | 91.4 | 89.7 |

These results showed that the DCs cultured according different variations of the method described in this patent are able to respond to physiological stimuli, such as CD40L by increasing their rate of surface expression of the loaded antigens, therefore incrementing their ability to induce a T-cell driven immune response against cells expressing the said antigens.

At harvesting, both batch 1 and 2 showed a mature phenotype, according to the expression of the standard markers for functional DCs, i.e. high expression of CD40, CD80, CD83, HLA-ABC and HLA-DR, coupled to low to no expression of CD14 (immature DCs) as well as of CD3 (T-cells) and CD19 (B-cells), used to determine the levels of a potential lymphocyte contamination. Accordingly, different loading methods and maturing schedules did not affect the phenotype of the final product, which appeared accomplishing the standards reported in literature (Nicolette et al., 2007).

The results obtained just upon a cycle of freezing/thawing showed that the final product remained stable according to the phenotype (see comparison between phenotype at harvesting and upon thawing regarding batch 2:
**a)** Detection of the Batch 1 DC phenotype between harvesting and 24h reculturing in plain medium

| **Batch 01** | | | | | |
|---|---|---|---|---|---|
| **Marker** | **At harvesting** | **Upon Thawing** | **Upon 4h Reculturing** | **Upon 12h Reculturing** | **Upon 24h Reculturing** |
| CD80 | 76.2 | n.a. | 86.9 | n.a. | 99.6 |
| CD83 | 73.7 | n.a. | 74.1 | n.a. | 94.6 |
| HLA-ABC | 89.5 | n.a. | 99.1 | n.a. | 99.9 |
| HLA-DR | 90 | n.a. | 98 | n.a. | 99 |
| CD40 | 88.6 | n.a. | 66 | n.a. | 98.7 |
| CD14 | 9.9 | n.a. | 10.4 | n.a. | 0.4 |
| CD19 | 1.5 | n.a. | 0.1 | n.a. | 0.6 |
| CD3 | 0.7 | n.a. | 0.2 | n.a. | 0.1 |

**b)** Detection of the Batch 2 DC phenotype between harvesting and 24h reculturing in presence of CD40L stimulation

| **Batch 02** | | | | | |
|---|---|---|---|---|---|
| **Marker** | **At harvesting** | **Upon Thawing** | **Upon 4h Reculturing** | **Upon 12h Reculturing** | **Upon 24h Reculturing** |
| CD80 | 72.4 | 98.7 | 88.4 | n.a. | 99.3 |
| CD83 | 81.8 | 81.8 | 64.4 | n.a. | 85.7 |
| HLA-ABC | 90.5 | 99.4 | 99.3 | n.a. | 100 |
| HLA-DR | 91.2 | 97.7 | 54.7 | n.a. | 99.9 |
| CD40 | 87.7 | 92 | 67.2 | n.a. | 98.9 |
| CD14 | 8 | 4.8 | 2.7 | n.a. | 0.7 |
| CD19 | 2.5 | 1 | 0.2 | n.a. | 0.7 |
| CD3 | 1.8 | 0.4 | 0.2 | n.a. | 0.5 |

**c)** Detection of the Batch 3 DC phenotype between harvesting and 24h reculturing in presence of CD40L stimulation

| **Batch 03** | | | | | |
|---|---|---|---|---|---|
| **Marker** | **At harvesting** | **Upon Thawing** | **Upon 4h Reculturing** | **Upon 12h Reculturing** | **Upon 24h Reculturing** |
| CD80 | n.a. | 34.2 | n.a. | 56.1 | 49.3 |
| CD83 | n.a. | 29.3 | n.a. | 59.7 | 44.6 |
| HLA-ABC | n.a. | 97.2 | n.a. | 98.8 | 95.8 |
| HLA-DR | n.a. | 57.1 | n.a. | 64.4 | 53.1 |
| CD40 | n.a. | 32.3 | n.a. | 98.2 | 46.5 |
| CD14 | n.a. | 18.3 | n.a. | 8.3 | 13.3 |
| CD19 | n.a. | 6.2 | n.a. | 7.3 | 5.4 |
| CD3 | n.a. | 39.7 | n.a. | 38.9 | 45.3 |

Interestingly, if the cells were transdifferentiated for 6 instead of 5 days, their phenotype correlated with a non-functional phenotype i.e. the surface expression of CD40, CD80 and CD83 did decrease to one third of the standard levels, while HLA-DR to about a half of them. Upon 24h reculturing, the phenotype of cells transdifferentiated for 5 days remained overall stable, while when they were transdifferentiated for 6 days, they increased mildly the levels of the mature phenotypic markers (Fig. 10), although remaining unable to reach standard levels of them. The results argued that longer than 5 days transdifferentiation, at least in DCs cultured according to the method depicted by this patent, may induce a non-functional phenotype, resulting in less DC activity (reduced expression of CD83) and reduced capability to dock the T-cells (reduced expression of CD80), accompanied by less efficient presentation to CD4' T-cells (reduced expression of HLA-DR. These feature are accompanied with altered morphology i.e. loss of cell adherence, compatible with less functional and less phenotypically matured DCs. Use of variations of the same method described in this patent did not affect the phenotypical maturation of the DCs, both providing state-of-art morphologically and phenotypically mature DCs.

Upon reculturing, the production of IL-10 and IL-12 increased, Interestingly, only the secretion of IL-12 seemed to be positively affected by CD40L, whereas the secretion of IL-10 was independent from this kind of stimulation. The IL-12 secretion was always at least double than that of IL-10. According to the publication of Lopez et al., 2005, such pattern would be more efficient in comparison to single IL-12 secretion to activate the immune system against cancer, due to its compatibility to activate both cytotoxic (with IL-12) and adaptive (with IL-10) responses:
**a)** Analysis of IL-10 secretion upon 24h reculturing in plain medium

| **IL-10: reculturing in plain medium** | | | |
|---|---|---|---|
| **Batch** | **Upon 4h Reculturing** | **Upon 12h Reculturing** | **Upon 24h Reculturing** |
| 01 | 3.87 | n.a. | 130.17 |
| 02 | 11.58 | n.a. | 240.17 |
| 03 | n.a. | n.d. | 15.74 |

**b)** Analysis of IL-10 secretion upon 24h reculturing in presence of CD40L stimulation

| **IL-10: reculturing in medium + CD40L** | | | |
|---|---|---|---|
| **Batch** | **Upon 4h Reculturing** | **Upon 12h Reculturing** | **Upon 24h Reculturing** |
| 01 | 3.34 | n.a. | 97.27 |
| 02 | 13.41 | n.a. | 143.33 |
| 03 | n.a. | n.d. | 3.61 |

**c)** Analysis of IL-12 secretion upon 24h reculturing in plain medium

| **IL-12: reculturing in plain medium** | | | |
|---|---|---|---|
| **Batch** | **Upon 4h Reculturing** | **Upon 12h Reculturing** | **Upon 24h Reculturing** |
| 01 | 268.47 | n.a. | 433.37 |
| 02 | 503.94 | n.a. | 826.81 |
| 03 | n.a. | n.d. | 17.77 |

**d)** Analysis of IL-12 secretion upon 24h reculturing in presence of CD40L stimulation

| **IL-12: reculturing in medium + CD40L** | | | |
|---|---|---|---|
| **Batch** | **Upon 4h Reculturing** | **Upon 12h Reculturing** | **Upon 24h Reculturing** |
| 01 | 347.06 | n.a. | 658.51 |
| 02 | 574.75 | n.a. | 865.89 |
| 03 | n.a. | n.d. | 10.18 |

On the contrary, cells transdifferentiated for 6 days became unable to secrete neither IL-10 nor IL-12, confirming the out-matured pattern, already observed regarding morphology and phenotype.

### Conclusions

The method described in the examples can be applied in different variations, according to the major aim. For example, when it is important to obtain a clinical grade preparation, as required for the use of the DCs as therapeutic tools, the protein antigen is likely constituted by a peptide, due to the fact that the costs and the availability of a GMP-conform peptide are more favourable than those for the corresponding GMP-protein. Each peptide, according to its length and its immunogenic my be administered in a differnet sequence regarding the coupled administration of the mRNA antigen and of the maturation cocktail. While in general, the loading of the mRNA antigen in combination with a protein antigen can be performed either on 5 days or 6 days cultured DCs, the specific contemporaneous use of TERT-mRNA and Survivin gives the best results with 5 days transdiffernetiated DCs.

The experiments performed until now showed that the use of specific antigen may require minor adjustments/variations of the protocol to obtain the best results.

## Claims

1. Dendritic cell loaded with at least one nucleic acid molecule encoding a tumor associated antigen protein or fragment thereof and at least one tumor associated antigen protein or fragment thereof.

2. Cell according to claim 1, **characterised in that** the nucleic acid molecule is a ribonucleic acid, preferably mRNA, and/or a desoxyribonucleic acid.

3. Cell according to claim 1 or 2, **characterised in that** the tumor associated antigen is selected from the group consisting of cancer type-specific tumor associated antigens, preferably carcinoembryonic antigen (CEA), mucin (MUC), prostate-specific membrane antigen (PSMA), BRCA and ras, and/or a universal tumor associated antigen.

4. Cell according to any one of claims 1 to 3, **characterised in that** the dendritic cells are of human or animal origin.

5. Cell according to any one of claims 1 to 4, **characterised in that** the dendritic cells are matured.

6. Method for manufacturing dendritic cells loaded with at least one nucleic acid molecule encoding a tumor associated antigen or fragment thereof and at least one protein molecule being a tumor associated antigen or fragment thereof, preferably according to any one of claims 1 to 5, comprising the steps of:
- providing dendritic cells; and
- loading said cells with at least one nucleic acid molecule encoding a tumor associated antigen or fragment thereof and at least one protein molecule being a tumor associated antigen or fragment thereof.

7. Method according to claim 6, **characterised in that** dendritic cells are provided by transdifferentiating monocytes into immature dendritic cells.

8. Method according to claim 7, **characterised in that** the immature dendritic cells are matured upon or after loading as well as during the protein antigen loading.

9. Dendritic cells obtainable by a method according to any one of claims 6 to 8.

10. Pharmaceutical preparation comprising dendritic cells according to any one of claims 1 to 5 or dendritic cells according to claim 9.

11. Preparation according to claim 9, **characterised in that** the preparation comprises further at least one pharmaceutical excipient, at least one immunostimmulatory agent, at least one adjuvant.

12. Use of dendritic cells according to any one of claims 1 to 5 or dendritic cells according to claim 9 for the manufacture of a vaccine for treating or preventing cancer in individuals.
